# EUROPÄISCHE PATENTSCHRIFT

(11) **EP 0 987 252 B1**
(45) Veröffentlichungstag und Bekanntmachung des Hinweises auf die Patenterteilung: **20.11.2002**
(21) Anmeldenummer: 99115418.8
(22) Anmeldetag: 04.08.1999
(51) Int. Cl.: C07D 221/20, H01L 51/00, H05B 33/00, G03G 5/00

(54) **Benzidinderivate sowie deren Herstellung und Verwendung**
Benzidine derivatives, their preparation and their use
Dérivés de la benzidine, leur préparation et leur utilisation

(30) Priorität: 17.08.1998 DE 19837198
(43) Veröffentlichungstag der Anmeldung: 22.03.2000
(73) Patentinhaber: Osram Opto Semiconductors GmbH & Co. OHG, 93049 Regensburg (DE)
(72) Erfinder: Kanitz, Andreas, Dr., 91333 Höchstadt (DE); Schumann, Jörg, 91056 Erlangen (DE)
(74) Vertreter: Epping, Wilhelm, Dipl.-Ing.

(56) Entgegenhaltungen:
- EP-A- 0 463 996
- DD-A- 227 434

## Beschreibung

Die Erfindung betrifft neue Benzidinderivate, ein Verfahren zu deren Herstellung und deren Verwendung.

Die Benzidinderivate (Benzidin = 4,4'-Diaminobiphenyl) nach der Erfindung sind anellierte, verbrückte Spiroverbindungen folgender Struktur:

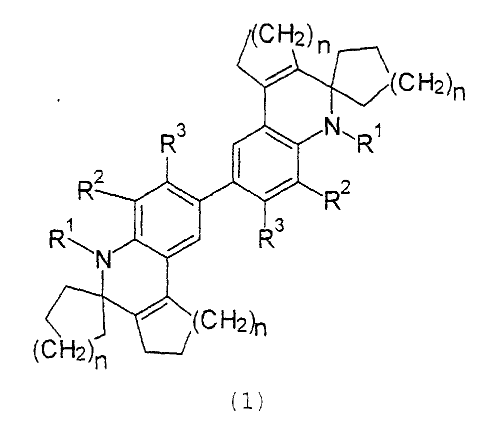

Dabei gilt folgendes:
n = 1, 2 oder 3;
R¹ = H, Phenyl oder 1-Naphthyl,
R² und R³ bilden zusammen einen anellierten Phenylring, einen a- oder b-anellierten Naphthylring oder einen anellierten 1,2,5-Thiadiazolring.
Vorzugsweise gilt folgendes:
n = 2,
R¹ = H oder Phenyl,
R² und R³ bilden zusammen einen anellierten Phenylring.

Die neuen Benzidinderivate sind sowohl anellierte, d.h. kondensierte Ringsysteme als auch verbrückte Spiroverbindungen, d.h. Verbindungen, bei denen ein Kohlenstoffatom, das sogenannte Spiroatom, zwei Ringen gemeinsam angehört. Außerdem sind in diesen Benzidinderivaten die Aminogruppierungen verbrückt, d.h. nicht mehr verdrillbar, so daß ein sehr starkes Donorsystem vorliegt.

Die Benzidinderivate (1) lassen sich in einfacher Weise aus leicht zugänglichen Ausgangsstoffen in hoher Reinheit herstellen. Dies geschieht in der Weise, daß ein aminosubstituierter Aromat oder Heteroaromat, d.h. eine heterocyclische Verbindung, der Struktur (2) und ein cycloaliphatisches Keton der Struktur (3), vorzugsweise im äquimolaren Verhältnis, ohne Zusatz eines Lösemittels in Gegenwart eines Katalysators, insbesondere einer katalytischen Menge Iod, bei erhöhter Temperatur miteinander zu einer verbrückten heterocyclischen Verbindung, d.h. einem aromatischen Amin, der Struktur (4) kondensiert werden; vorzugsweise erfolgt die Umsetzung der beiden Edukte bei einer Temperatur zwischen 200 und 250°C.

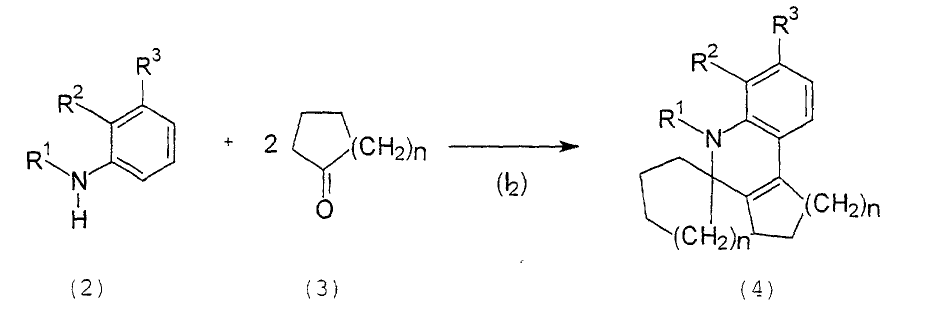

Das Kondensationsprodukt (4), das stark nucleophil ist, wird dann zum Benzidinderivat (1) oxidiert. Dies erfolgt vorzugsweise mit Wasserstoffperoxid in einem polaren Lösemittel, wie Dimethylformamid. Zur Oxidation können auch Sauerstoff und andere Oxidationsmittel eingesetzt werden. Bei der Oxidation mit Wasserstoffperoxid ist es von Vorteil, wenn ein Kupfersalz, wie Kupfer(I)-chlorid (CuCl), zugegeben wird.

Das Benzidinderivat (1) wird auch dann erhalten, wenn das Kondensationsprodukt (4) - nach seiner Bildung - durch Destillation im Feinvakuum abgetrennt wird; unter diesen Bedingungen wird es nämlich - aufgrund seiner hohen Nucleophilie - zum Benzidinderivat oxidiert. Das bei der Destillation anfallende honigartige Produkt besteht im wesentlichen aus einem Gemisch von (1) und (4), das mit Diethylether leicht getrennt werden kann. Das Benzidinderivat (1) ergibt dabei einen flockigen Niederschlag, während das Kondensationsprodukt (4) in Lösung bleibt.

Auf die beschriebene Weise können Verbindungen (1) mit R¹ = H, Phenyl oder 1-Naphthyl hergestellt werden. Die Phenylund Naphthylverbindungen können aber auch in der Weise hergestellt werden, daß die entsprechende H-Verbindung noch aryliert wird. Die Arylierung erfolgt dabei in an sich bekannter Weise durch eine der Ullmann-Reaktion bzw. der Heck-Reaktion analoge Umsetzung, beispielsweise durch Reaktion mit Brombenzol in Gegenwart eines Palladiumkatalysators.

Die Verbindungen der Struktur (1) sind - aufgrund der Spiro-Struktur - schwer kristallisierbar; die in Form amorpher Pulver anfallenden Verbindungen weisen eine Glasübergangstemperatur > 220°C auf.

Die Verbindungen nach der Erfindung sind fluoreszierende Chromophore. Da diese Verbindungen - infolge der eingeschränkten Verdrillungsmöglichkeit des Stickstoffs und der Anellierung - leichter oxidierbar sind als bislang bekannte Benzidinderivate, eignen sie sich in hervorragender Weise als Lochtransportmaterialien in organischen Leuchtdioden. Lochtransportmaterialien auf der Basis von Tetraarylbenzidin sind beispielsweise in der Dissertation von J. Simmerer "Ladungstransport in kolumnaren Phasen", Bayreuth 1996, beschrieben (Seiten 4 bis 10); siehe dazu auch: "Molecular Crystals and Liquid Crystals", Vol. 183 (1990), Seiten 217 bis 226, "Applied Physics Leiters", Vol. 65 (1994), Seiten 807 bis 809, und "Chemical Physics", Vol. 200 (1995), Seiten 245 bis 255.

Aufgrund des Vorhandenseins anellierter Strukturelemente wird zusätzlich die langwelligste Absorption der Verbindungen (1) in den sichtbaren Spektralbereich verschoben. Dadurch können diese Verbindungen - je nach Anellierungstyp - in verschiedenen Bereichen des sichtbaren Spektralbereiches emittieren, wodurch sie gleichzeitig als Emitterchromophor fungieren können. Dagegen absorbieren und emittieren die bislang bekannten Materialien ausschließlich im UV-Bereich (des elektromagnetischen Spektrums).

Die Verbindungen nach der Erfindung finden insbesondere in organischen Leuchtdioden Verwendung, d.h. in organischen Licht-emittierenden Dioden (OLEDs), sowie in damit hergestellten Pixeldisplays für Informationsbildschirme und Lichtquellen. Im Vergleich zu den bislang verwendeten Materialien (auf der Basis von Tetraarylbenzidin) zeigen die Benzidinderivate nach der Erfindung verbesserte Materialeigenschaften. Dies sind insbesondere eine höhere Glasübergangstemperatur, was eine höhere thermische Stabilität der Leuchtdioden zur Folge hat, und eine geringere Potentialdifferenz zwischen dem Material und der ITO-Elektrode (ITO = Indium-Tin-Oxide), was zu einer höheren Lichtausbeute der Leuchtdioden führt. Bei den bislang eingesetzten Materialien liegen nämlich die HOMO-Potentiale gegenüber dem Potential der ITO-Elektrode energetisch tiefer, was sich in einer geringeren Lichtausbeute auswirkt.

Anhand von Ausführungsbeispielen soll die Erfindung noch näher erläutert werden.

### Beispiel 1

In einem 0,5 l-Dreihalskolben, der mit einem Rückflußkühler, einer Destillationsapparatur und einem Tropftrichter (mit einem bis zum Kolbenboden reichenden Rohr) versehen ist, werden 0,5 mol 1-Naphthylamin mit ca. 1,5 g Iod auf 200°C erhitzt; anschließend werden 1,5 mol Cyclohexanon tropfenweise zugefügt. Das bei der Reaktion gebildete Wasser wird aus dem Reaktionskolben durch Destillation entfernt. Sobald das Wasser abdestilliert ist, wird im Feinvakuum rektifiziert (Sdp. bei 6·10⁻⁵ Torr: 150 bis 180°C). Das dabei erhaltene ölige Produkt wird mit Diethylether digeriert, wobei das Benzidinderivat in gelblichen Flocken ausfällt, die durch Absaugen abgetrennt werden.

### Beispiel 2

Es wird zunächst entsprechend Beispiel 1 vorgegangen. Wenn das Wasser entfernt ist, läßt man auf 120°C abkühlen, und dann werden zum Reaktionsgemisch 200 ml Dimethylformamid (als Lösungsvermittler) und 0,1 g Kupfer(I)-chlorid gegeben. Anschließend werden durch den Rückflußkühler 60 g 30 %iges Wasserstoffperoxid zugetropft. Nach erfolgter Umsetzung werden das dabei gebildete Wasser und das Dimethylformamid durch Destillation entfernt, und dann wird im Feinvakuum rektifiziert (Sdp. bei 6·10⁻⁵ Torr: 150 bis 180°C). Das dabei erhaltene ölige Produkt wird mit Diethylether digeriert, wobei das Benzidinderivat in gelblichen Flocken ausfällt, die durch Absaugen abgetrennt werden. Das erhaltene Produkt kann zur weiteren Reinigung noch sublimiert werden.

### Beispiel 3

In einem 0,5 l-Dreihalskolben, der mit einem Rückflußkühler, einer Destillationsapparatur und einem Tropftrichter (mit einem bis zum Kolbenboden reichenden Rohr) versehen ist, werden 0,5 mol N-Phenyl-1-naphthylamin mit ca. 1,5 g Iod auf 200°C erhitzt; anschließend werden 1,5 mol Cyclohexanon tropfenweise zugefügt. Das bei der Reaktion gebildete Wasser wird aus dem Reaktionskolben durch Destillation entfernt. Danach wird wie in Beispiel vorgegangen, wobei der Siedepunkt bei der Rektifizierung im Feinvakuum (6·10⁻⁵ Torr) 220 bis 240°C beträgt.

### Beispiel 4

0,05 mol der entsprechend Beispiel 1 oder 2 erhaltenen Verbindung und 0,05 mol Brombenzol werden in wasserfreiem und mittels Inertgas gespültem Toluol gelöst und mit 3 Mol-% eines Palladiumkatalysators versetzt, der aus Tri-o-tolylphosphin und Palladium(II)-acetat hergestellt wurde. Die Reaktionslösung wird anschließend mit 0,06 mol Natrium-tert.butoxid versetzt und 15 h unter Rückfluß erhitzt. Das dabei erhaltene Produkt wird dünnschichtchromatographisch abgetrennt und dann sublimiert. Es wird die gleiche Verbindung erhalten wie bei Beispiel 3.

## Patentansprüche

1. Benzidinderivate der Struktur 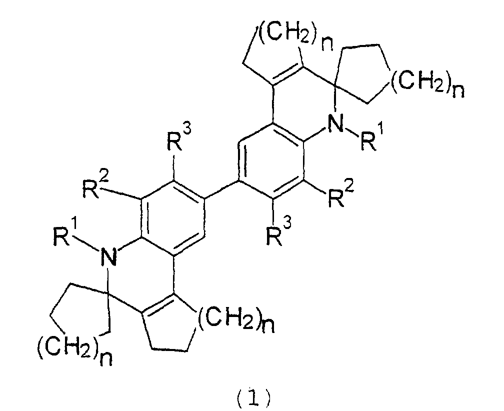 wobei folgendes gilt:
n = 1, 2 oder 3;
R¹ = H, Phenyl oder 1-Naphthyl,
R² und R³ bilden zusammen einen anellierten Phenylring, einen a- oder b-anellierten Naphthylring oder einen anellierten 1,2,5-Thiadiazolring.

2. Benzidinderivate nach Anspruch 1, **dadurch gekennzeichnet, daß** folgendes gilt:
n = 2,
R¹ = H oder Phenyl,
R² und R³ bilden zusammen einen anellierten Phenylring.

3. Verfahren zur Herstellung von Benzidinderivaten nach Anspruch 1 oder 2, **dadurch gekennzeichnet, daß** eine Verbindung der Struktur 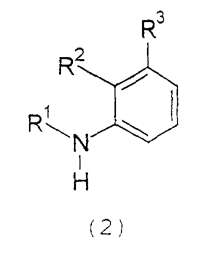 mit einer Verbindung der Struktur 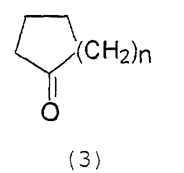 bei erhöhter Temperatur in Gegenwart eines Katalysators zu einer Verbindung der Struktur 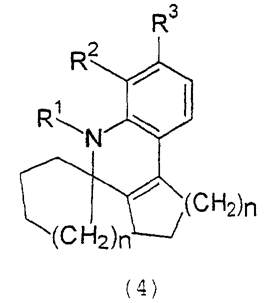 umgesetzt wird, daß die Verbindung (4) durch Oxidation in ein Benzidinderivat (1) übergeführt wird, und daß das Benzidinderivat (1) gegebenenfalls aryliert wird.

4. Verfahren nach Anspruch 3, **dadurch gekennzeichnet, daß** der Katalysator Iod ist.

5. Verfahren nach Anspruch 3 oder 4, **dadurch gekennzeichnet, daß** die Umsetzung bei einer Temperatur zwischen 200 und 250°C durchgeführt wird.

6. Verfahren nach einem der Ansprüche 3 bis 5, **dadurch gekennzeichnet, daß** zur Oxidation Wasserstoffperoxid verwendet wird.

7. Verfahren nach Anspruch 6, **dadurch gekennzeichnet, daß** ein Kupfersalz als Katalysator zugesetzt wird.

8. Verfahren nach Anspruch 6 oder 7, **dadurch gekennzeichnet, daß** die Oxidation in einem polaren Lösemittel erfolgt.

9. Verfahren nach einem oder mehreren der Ansprüche 3 bis 8, **dadurch gekennzeichnet, daß** die Arylierung mit Brombenzol in Gegenwart eines Palladiumkatalysators erfolgt.

10. Verwendung der Benzidinderivate nach Anspruch 1 oder 2 als Lochtransportmaterialien in organischen Leuchtdioden.

## Claims

1. Benzidine derivatives of the structure 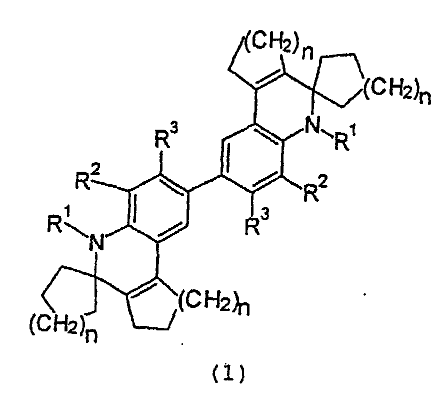 in which:
n = 1, 2 or 3;
R¹ = H, phenyl or 1-naphthyl, and
R² and R³ together form a fused phenyl ring, an a-or b-fused naphthyl ring or a fused 1,2,5-thiadiazole ring.

2. Benzidine derivatives as claimed in claim 1, **characterized in that** n = 2, R¹ = H or phenyl, and R² and R³ together form a fused phenyl ring.

3. A process for the preparation of benzidine derivatives as claimed in claim 1 or 2, **characterized in that** it comprises a compound of the structure 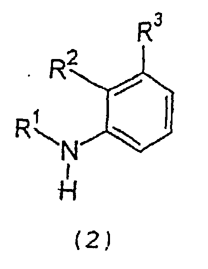 with a compound of the structure 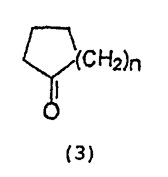 at an elevated temperature in the presence of a catalyst, to give a compound of the structure 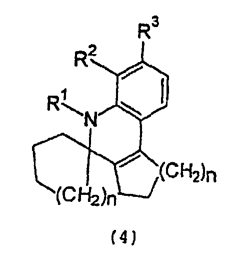 and converting the compound (4) into a benzidine derivative (1) by oxidation, and **in that** the benzidine derivative (1) is optionally arylated.

4. A process as claimed in claim 3, **characterized in that** the catalyst is iodine.

5. A process as claimed in claim 3 or 4, **characterized in that** the reaction is carried out at a temperature of from 200 to 250°C.

6. A process as claimed in any of claims 3 to 5, **characterized in that** the oxidation is carried out using hydrogen peroxide.

7. A process as claimed in claim 6, **characterized in that** a copper salt is added as catalyst.

8. A process as claimed in claim 6 or 7, **characterized in that** the oxidation is carried out in a polar solvent.

9. A process as claimed in one or more of claims 3 to 8, **characterized in that** the arylation is carried out using bromobenzene in the presence of a palladium catalyst.

10. Use of the benzidine derivatives according to claim 1 or 2 as hole transport materials in organic light emitting diodes.

## Revendications

1. Dérivé de la benzidine de formule développée 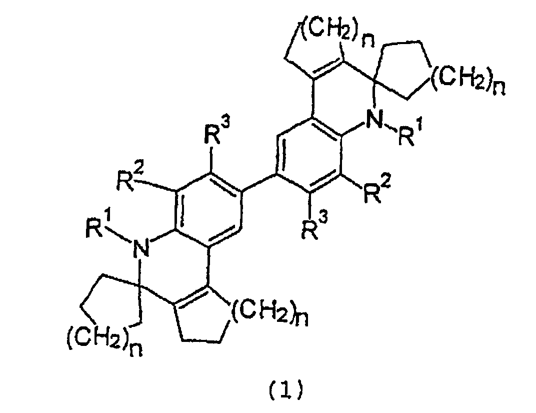 dans laquelle :
n = 1, 2 ou 3 ;
R¹ = H, phényle ou 1-naphtyle,
R² et R³ forment ensemble un cycle phényle condensé, un cycle naphtyle condensé en a ou condensé en b, ou un cycle 1,2,5-thiadiazol condensé.

2. Dérivé de la benzidine suivant la revendication 1, **caractérisé en ce que** :
n = 2,
R¹ = H ou phényle,
R² et R³ forment ensemble un cycle phényle condensé.

3. Procédé de préparation de dérivé de la benzidine suivant la revendication 1 ou 2, **caractérisé en ce que** l'on met un composé de formule développée 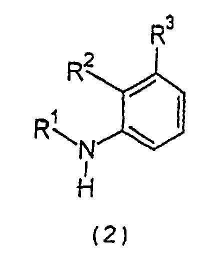 à réagir sur un composé de formule développée 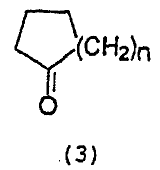 à haute température en présence d'un catalyseur pour obtenir un composé de formule développée 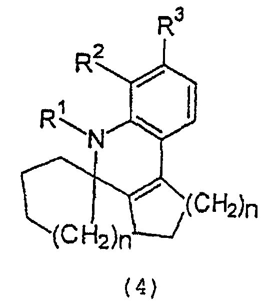 **en ce que** l'on transforme le composé (4) par oxydation en un dérivé (1) de la benzidine, et **en ce que**, le cas échéant, on condense le dérivé (1) de la benzidine.

4. Procédé suivant la revendication 3, **caractérisé en ce que** le catalyseur est de l'iode.

5. Procédé suivant la revendication 3 ou 4, **caractérisé en ce que** l'on effectue la réaction à une température comprise entre 200 et 250°C.

6. Procédé suivant l'une des revendications 3 à 5, **caractérisé en ce que** l'on utilise pour l'oxydation de l'eau oxygénée.

7. Procédé suivant la revendication 6, **caractérisé en ce que** l'on ajoute comme catalyseur un sel de cuivre.

8. Procédé suivant la revendication 6 ou 7, **caractérisé en ce que** l'on effectue l'oxydation dans un solvant polaire.

9. Procédé suivant l'une ou plusieurs des revendications 3 à 8, **caractérisé en ce que** l'on effectue la condensation par du bromobenzène en présence d'un catalyseur au palladium.

10. Utilisation des dérivés de la benzidine suivant la revendication 1 ou 2 comme matériaux de transport de trous dans des diodes luminescentes organiques.
